# EUROPEAN PATENT APPLICATION

(11) **EP 1 500 642 A1**
(43) Date of publication of application: **26.01.2005**
(21) Application number: 03719137.6
(22) Date of filing: 18.04.2003
(51) Int. Cl.: C07C 69/96, C07C 68/02

(54) **3-SUBSTITUTED OXYGLUTARIC DIESTER COMPOUND, OPTICALLY ACTIVE 3-SUBSTITUTED OXYGLUTARIC MONOESTER COMPOUND, AND PROCESSES FOR PRODUCING THESE**

(30) Priority: 19.04.2002 JP 2002117285; 19.04.2002 JP 2002117286
(71) Applicant: Ube Industries, Ltd., Ube-Shi, Yamaguchi 755-8633 (JP)
(72) Inventor: YAMAMOTO, Y., c/o Ube Research Laboratory, Ube-shi, Yamaguchi 755-8633 (JP); MIYATA, H., c/o Ube Research Laboratory, Ube-shi, Yamaguchi 755-8633 (JP); KONEGAWA, T., c/o Ube Research Laboratory, Ube-shi, Yamaguchi 755-8633 (JP); SAKATA, K., c/o Ube Research Laboratory, Ube-shi, Yamaguchi 755-8633 (JP)
(74) Representative: Becker, Philippe
(86) International application number: PCT/JP2003/004962
(87) International publication number: WO 2003/089401

(57) **Abstract**

The present invention is to provide a 3-substituted oxyglutaric acid diester compound represented by the following formula (I): 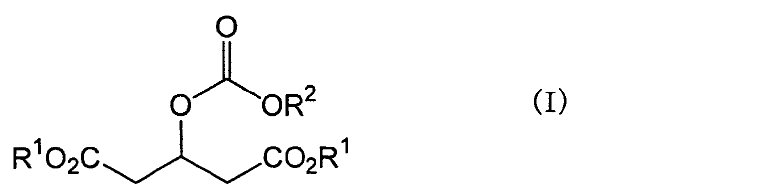 wherein R¹ may be the same or different from each other, and represents a substituted or unsubstituted alkyl group, R² represents a substituted or unsubstituted alkyl group, a substituted or unsubstituted alkenyl group, a substituted or unsubstituted aralkyl group or a substituted or unsubstituted aryl group,
and an optically active 3-substituted oxyglutaric acid monoester compound represented by the following formula (IV) : 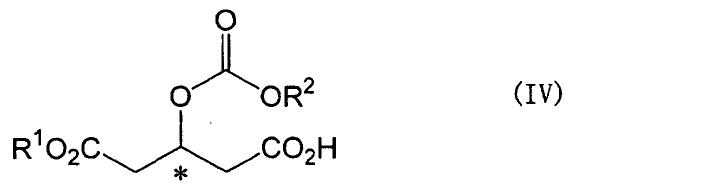 wherein R¹ and R² have the same meanings as defined above,
and processes for preparing the same.

## Description

### Technical field

The present invention relates to a novel 3-substituted oxyglutaric acid diester compound and optically active 3-substituted oxyglutaric acid monoester compound, and processes for preparing these. The 3-substituted oxyglutaric acid diester compound of the present invention can be easily led to an optically active 3-hydroxyglutarate monoester which is useful as a synthetic intermediate for a medicine by hydrolyzing one of ester groups to prepare an optically active 3-substituted glutaric acid monoester compound, and then, subjecting to conventionally known reducing method (shown in Reference example 1 mentioned below).

### Background art

The 3-substituted oxyglutaric acid diester compound of the present invention is a novel compound, and a process for preparing the same has never been known as of today.

On the other hand, a method for obtaining an optically active 3-substituted oxyglutaric acid monoester compound by selectively hydrolyzing one of ester groups of a 3-substituted oxyglutaric acid diester compound using a hydrolase has been disclosed in the following references.
① Canadian Journal of Chemistry (Can. J. Chem.), 66, 1422 (1988); a method for producing an optically active 3-alkoxyglutaric acid monoester by hydrolyzing a 3-alkoxyglutaric acid diester in a buffer solution in the presence of an esterase originated from pig liver is disclosed. However, in this method, there are problems that an amount of the enzyme to be used is too much, and an optical purity of the objective compound is low.
② Tetrahedron Letters (Tetrahedron Lett.), 28, 4935 (1987); a method for producing an optically active 3-(R)-alkoxyglutaric acid monomethyl ester and a 3-(R)-acyloxy-glutaric acid monomethyl ester by acting α-chymotrypsin on a 3-alkoxyglutaric acid dimethyl ester and a 3-acyloxy-glutaric acid dimethyl ester in a buffer to carry out hydrolysis is disclosed. However, in this method, there are problems that an amount of oxygen to be used is too much, and an optical purity of the objective compound is low.
③ Journal of Organic Chemistry (J. Org. Chem.), 61,6024 (1996); a method for producing an optically active 3-(S)-acetyloxyglutaric acid monoester by hydrolyzing a 3-acetyl-oxymonoglu acid diester in a mixed solvent of a phosphate buffer with a pH of 7 and 1,4-dioxane in the presence of a lipase originated from *Candida Antarctica* is disclosed. However, in this method, there are problems that an amount of oxygen to be used is too much, an optical purity of the objective compound is low and high carcinogenic 1,4-dioxane must be used with a large amount.

In either of the above-mentioned methods, various problems are involved, and they are not effective as an industrial process for producing an optically active 3-substituted oxyglutaric acid monoester compound.

An object of the present invention is to provide a novel 3-substituted oxyglutaric acid diester compound and a process for preparing the same.

Another object of the present invention is to solve the above-mentioned problems, and to provide an optically active 3-substituted oxyglutaric acid monoester compound which is industrially suitable with high yield and high selectivity and a process for preparing the same from a 3-substituted oxyglutaric acid diester compound with a simple and easy method.

### Disclosure of the invention

An object of the present invention can be accomplished by a 3-substituted oxyglutaric acid diester compound represented by the formula (I): 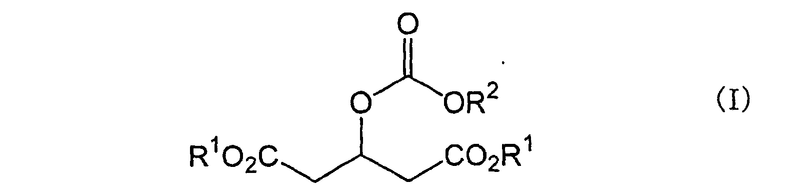 wherein R¹ may be the same or different from each other, and represents a substituted or unsubstituted alkyl group, R² represents a substituted or unsubstituted alkyl group, a substituted or unsubstituted alkenyl group, a substituted or unsubstituted aralkyl group or a substituted or unsubstituted aryl group.

An object of the present invention can be also accomplished by a process for preparing a 3-substituted oxyglutaric acid diester compound which comprises reacting a 3-hydroxyglutaric acid diester represented by the formula (II): 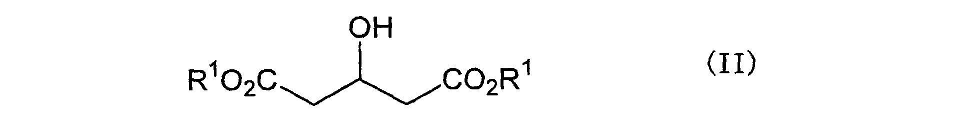 wherein R¹ has the same meaning as defined above, and a halogenoformate represented by the formula (III): 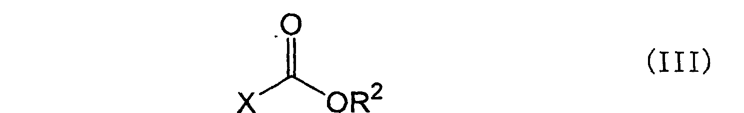 wherein X represents a halogen atom, R² has the same meaning as defined above,
in the presence of a base.

An object of the present invention can be also accomplished by an optically active 3-substituted oxyglutaric acid monoester compound represented by the formula (IV): 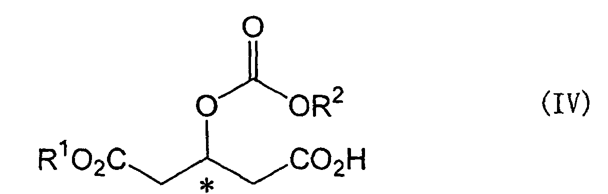 wherein R¹ and R² have the same meanings as defined above.

An object of the present invention can be further accomplished by a process for preparing an optically active 3-substituted oxyglutaric acid monoester compound which comprises selectively hydrolyzing one of ester groups of a 3-substituted oxyglutaric acid diester compound represented by the above-mentioned formula (I): 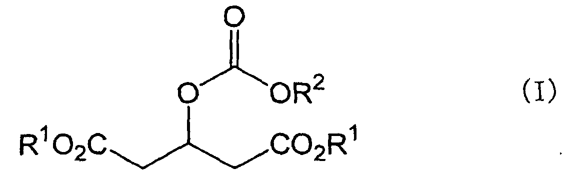 wherein R¹ and R² have the same meanings as defined above,
in the presence of a hydrolase.

### Best mode for carrying out the invention

The 3-substituted oxyglutaric acid diester compound in the present invention is represented by the above-mentioned formula (I). In the formula (I), R¹ represents a substituted or unsubstituted alkyl group which may be the same or different from each other.

The above-mentioned substituted or unsubstituted alkyl group means (1) "an alkyl group having no substituent" or (2) "an alkyl group having a substituent(s)". As "the alkyl group having no substituent" of (1), there may be mentioned, for example, an alkyl group having 1 to 10 carbon atoms such as a methyl group, an ethyl group, a propyl group, a butyl group, a pentyl group, a hexyl group, a heptyl group, an octyl group, a nonyl group, a decyl group, etc. (incidentally, these groups may include various kinds of isomers), preferably a methyl group, an ethyl group, a n-propyl group, an isopropyl group, a n-butyl group, a t-butyl group, an n-octyl group, more preferably a methyl group, an ethyl group. As the substituent for "the alkyl group having a substituent(s)" of (2), there may be mentioned, for example, a halogen atom such as a fluorine atom, a chlorine atom, a bromine atom, an iodine atom, etc.; a hydroxyl group; an alkoxyl group having 1 to 4 carbon atoms such as a methoxyl group, an ethoxyl group, a propoxyl group, a butoxyl group, etc. (incidentally, these groups may include various kinds of isomers.); a dialkylamino group such as a dimethylamino group, diethylamino group, etc. (incidentally, these groups may include various kinds of isomers.); a cyano group, etc., preferably a fluorine atom, a chlorine atom, a hydroxyl group, cyano group. As the alkyl group having a substituent(s), there may be specifically mentioned a 2-chloroethyl group, a 2,2-dichloroethyl group, a 2,2,2-trichloroethyl group, a 2,2,2-trifluoroethyl group, a 2-hydroxyethyl group, a 2-cyanoethyl group, a 2-methoxyethyl group, a 2-ethoxyethyl group, a 2-bromoethyl group, a 2-dimethylamino group, a 2-chloropropyl group, a 3-chloropropyl group, etc., preferably a 2-chloroethyl group, a 2,2,2-trichloroethyl group, a 2,2,2-trifluoroethyl group or a 2-cyanoethyl group.

R² of Compound (I) represents a substituted or unsubstituted alkyl group, a substituted or unsubstituted alkenyl group, a substituted or unsubstituted aralkyl group or a substituted or unsubstituted aryl group.

The substituted or unsubstituted alkyl group of the above-mentioned R² has the same meaning as the substituted or unsubstituted alkyl group of the above-mentioned R¹.

The substituted or unsubstituted alkenyl group of the above-mentioned R² is (3) "an alkenyl group having no substituent " or (4) "an alkenyl group having a substituent(s)". As "the alkenyl group having no substituent" of (3), there may be mentioned, for example, an alkenyl group having 2 to 10 carbon atoms such as a vinyl group, a propenyl group, a butenyl group, a pentenyl group, a hexenyl group, a heptenyl group, an octenyl group, a nonenyl group, a decenyl group, etc. Incidentally, these groups may include various kinds of isomers. As the substituent for "the alkenyl group having a substituent(s)" of (4), there may be mentioned, for example, a vinyl group and a propenyl group.

The substituted or unsubstituted aralkyl group of the above-mentioned R² is (5) "an aralkyl group having no substituent" or (6) "an aralkyl group having a substituent(s)". As "the aralkyl group having no substituent" of (5), there may be mentioned, for example, an aralkyl group (incidentally, these groups may include various kinds of isomers.) such as a benzyl group, a phenethyl group, etc., and a benzyl group is particularly preferred. As the substituent for "the aralkyl group having a substituent(s)" of (6), there may be mentioned, for example, a hydroxyl group; a nitro group; a halogen atom such as a fluorine atom, a chlorine atom, a bromine atom, an iodine atom, etc.; an alkoxyl group having 1 to 10 carbon atoms (incidentally, these groups may include various kinds of isomers.) such as a methoxyl group, an ethoxyl group, a propoxyl group, a butoxyl group, a pentyloxyl group, a hexyloxyl group, a heptyloxyl group, an octyloxyl group, a nonyloxyl group, a decyloxyl group, etc.; an aralkyloxyl group having 7 to 10 carbon atoms (incidentally, these groups may include various kinds of isomers.) such as a benzyloxyl group, a phenethyloxyl group, etc.; an aryloxyl group having 6 to 10 carbon atoms (incidentally, these groups may include various kinds of isomers) such as a phenyloxyl group, etc.; an alkoxyalkoxyl group having 2 to 10 carbon atoms (incidentally, these groups may include various kinds of isomers.) such as a methoxymethoxyl group, etc.; a monoalkylamino group (incidentally, these groups may include various kinds of isomers.) such as a methylamino group, an ethylamino group, etc.; an alkylamino group (incidentally, these groups may include various kinds of isomers) such as a dimethylamino group, etc.; an acylamino group such as a formylamino group, an acetylamino group, a benzoylamino group, etc.

The substituted or unsubstituted aryl group of the above-mentioned R² is (7) "an aryl group having no substituent" or (8) "an aryl group having a substituent(s)". As "the aryl group having no substituent" of (7), there may be mentioned, for example, a phenyl group, a naphthyl group, an anthracenyl group, a thienyl group, etc., preferably a phenyl group, a naphthyl group, more preferably a phenyl group. As the substituent for "the aryl group having a substituent(s)" of (8), there may be mentioned, for example, a hydroxyl group; a nitro group; a halogen atom such as a fluorine atom, a chlorine atom, a bromine atom, an iodine atom, etc.; an alkoxyl group having 1 to 10 carbon atoms (incidentally, these groups may include various kinds of isomers.) such as a methoxyl group, an ethoxyl group, a propoxyl group, a butoxyl group, a pentyloxyl group, a hexyloxyl group, a heptyloxyl group, an octyloxyl group, a nonyloxyl group, a decyloxyl group, etc.; an aralkyloxyl group having 7 to 10 carbon atoms (incidentally, these groups may include various kinds of isomers.) such as a benzyloxyl group, a phenethyloxyl group, etc.; an aryloxyl group having 6 to 10 carbon atoms (incidentally, these groups may include various kinds of isomers.) such as a phenyloxyl group, etc.; an alkoxyalkoxyl group having 2 to 10 carbon atoms (incidentally, these groups may include various kinds of isomers.) such as a methoxymethoxyl group, etc.; a monoalkylamino group (incidentally, these groups may include various kinds of isomers.) such as a methylamino group, an ethylamino group, etc.; a dialkylamino group (incidentally, these groups may include various kinds of isomers.) such as a dimethylamino group, etc.; an acylamino group such as a formylamino group, an acetylamino group, a benzoylamino group, etc.

Of these R², preferred is a group selected from the group consisting of a benzyl group, a 2-methylbenzyl group, a 3-methylbenzyl group, a 4-methylbenzyl group, a 2-methoxybenzyl group, a 3-methoxybenzyl group, a 4-methoxybenzyl group, a 2-chlorobenzyl group, a 3-chlorobenzyl group, a 4-chlorobenzyl group, a 2-bromobenzyl group, a 3-bromobenzyl group, a 4-bromobenzyl group, a 2-fluorobenzyl group, a 3-fluorobenzyl group, a 4-fluorobenzyl group, a 2-nitrobenzyl group, a 3-nitrobenzyl group, a 4-nitrobenzyl group, a 2-methoxybenzyl group, a 3-methoxybenzyl group, a 4-methoxybenzyl group, a t-butyl group, a methyl group, an isopropyl group, a phenyl group, a vinyl group and an allyl group, and a benzyl group is particularly preferred.

As the specific examples of the 3-substituted oxyglutaric acid diester compound having the above-mentioned R¹ and R², there may be mentioned, for example,
dimethyl 3-benzyloxycarbonyloxyglutarate,
diethyl 3-benzyloxycarbonyloxyglutarate,
dipropyl 3-benzyloxycarbonyloxyglutarate,
dibutyl 3-benzyloxycarbonyloxyglutarate,
dipentyl 3-benzyloxycarbonyloxyglutarate,
dihexyl 3-benzyloxycarbonyloxyglutarate,
diheptyl 3-benzyloxycarbonyloxyglutarate,
dioctyl 3-benzyloxycarbonyloxyglutarate,
dinonyl 3-benzyloxycarbonyloxyglutarate,
didecyl 3-benzyloxycarbonyloxyglutarate,
diisopropyl 3-benzyloxycarbonyloxyglutarate,
di-s-butyl 3-benzyloxycarbonyloxyglutarate,
di-t-butyl 3-benzyloxycarbonyloxyglutarate,
dichloromethyl 3-benzyloxycarbonyloxyglutarate,
dibenzyl 3-benzyloxycarbonyloxyglutarate,
di-4-nitrobenzyl 3-benzyloxycarbonyloxyglutarate,
di-4-trifluoromethylbenzyl 3-benzyloxycarbonyloxyglutarate,
di-4-chlorobenzyl 3-benzyloxycarbonyloxyglutarate,
di-4-bromobenzyl 3-benzyloxycarbonyloxyglutarate,
di-4-fluorobenzyl 3-benzyloxycarbonyloxyglutarate,
di-4-methoxybenzyl 3-benzyloxycarbonyloxyglutarate,
divinyl 3-benzyloxycarbonyloxyglutarate,
diallyl 3-benzyloxycarbonyloxyglutarate,
dimethyl 3-(2-methylbenzyl)oxycarbonylglutarate,
dimethyl 3-(3-methylbenzyl)oxycarbonylglutarate,
dimethyl 3-(4-methylbenzyl)oxycarbonylglutarate,
dimethyl 3-(2-methoxybenzyl)oxycarbonylglutarate,
dimethyl 3-(3-methoxybenzyl)oxycarbonylglutarate,
dimethyl 3-(4-methoxybenzyl)oxycarbonylglutarate,
dimethyl 3-(2-chlorobenzyl)oxycarbonylglutarate,
dimethyl 3-(3-chlorobenzyl)oxycarbonylglutarate,
dimethyl 3-(4-chlorobenzyl)oxycarbonylglutarate,
dimethyl 3-(2-bromobenzyl)oxycarbonylglutarate,
dimethyl 3-(3-bromobenzyl)oxycarbonylglutarate,
dimethyl 3-(4-bromobenzyl)oxycarbonylglutarate,
dimethyl 3-(2-fluorobenzyl)oxycarbonylglutarate,
dimethyl 3-(3-fluorobenzyl)oxycarbonylglutarate,
dimethyl 3-(4-fluorobenzyl)oxycarbonylglutarate,
dimethyl 3-(2-nitrobenzyl)oxycarbonylglutarate,
dimethyl 3-(3-nitrobenzyl)oxycarbonylglutarate,
dimethyl 3-(4-nitrobenzyl)oxycarbonylglutarate,
dimethyl 3-(2-methoxybenzyl)oxycarbonylglutarate,
dimethyl 3-(3-methoxybenzyl)oxycarbonylglutarate,
dimethyl 3-(4-methoxybenzyl)oxycarbonylglutarate,
dimethyl 3-(t-butoxycarbonyl)oxyglutarate,
dimethyl 3-methoxycarbonylglutarate,
dimethyl 3-isopropoxyoxyglutarate,
dimethyl 3-phenoxycarbonyloxyglutarate,
dimethyl 3-vinyloxyoxyglutarate,
dimethyl 3-allyloxyoxyglutarate,
etc., preferably
dimethyl 3-benzyloxycarbonyloxyglutarate,
diethyl 3-benzyloxycarbonyloxyglutarate.

The 3-hydroxyglutaric acid diester to be used in the reaction of the present invention is represented by the above-mentioned formula (II). In the formula (II), R¹ has the same meaning as defined above.

As the specific examples of the 3-hydroxyglutaric acid diester compound having the above-mentioned R¹, there may be mentioned, for example,
dimethyl 3-hydroxyglutarate,
diethyl 3-hydroxyglutarate,
dipropyl 3-hydroxyglutarate,
dibutyl 3-hydroxyglutarate,
dipentyl 3-hydroxyglutarate,
dihexyl 3-hydroxyglutarate,
diheptyl 3-hydroxyglutarate,
dioctyl 3-hydroxyglutarate,
dinonyl 3-hydroxyglutarate,
didecyl 3-hydroxyglutarate,
diisopropyl 3-hydroxyglutarate,
di-s-butyl 3-hydroxyglutarate,
di-t-butyl 3-hydroxyglutarate,
dichloromethyl 3-hydroxyglutarate,
dibenzyl 3-hydroxyglutarate,
di-4-nitrobenzyl 3-hydroxyglutarate,
di-4-trifluoromethylbenzyl 3-hydroxyglutarate,
di-4-chlorobenzyl 3-hydroxyglutarate,
di-4-bromobenzyl 3-hydroxyglutarate,
di-4-fluorobenzyl 3-hydroxyglutarate,
di-4-methoxybenzyl 3-hydroxyglutarate,
divinyl 3-hydroxyglutarate,
diallyl 3-hydroxyglutarate,
etc., preferably
dimethyl 3-hydroxyglutarate,and
diethyl 3-hydroxyglutarate
are used.

The halogenoformate to be used in the reaction of the present invention is represented by the above-mentioned formula (III). In the formula (III), X is a halogen atom such as a fluorine atom, a chlorine atom, a bromine atom, an iodine atom, etc., and R² has the same meaning as defined above.

As the specific examples of the halogenoformate having the above-mentioned X and R², there may be mentioned, for example,
methyl chloroformate,
ethyl chloroformate,
propyl chloroformate,
isobutyl chloroformate,
(2-bromoethyl)chloroformate,
(2,2,2-trichloroethyl)chloroformate,
phenyl chloroformate,
(p-tolyl) chloroformate,
nitrophenyl chloroformate,
(p-chlorophenyl) chloroformate,
vinyl chloroformate,
allyl chloroformate,
benzyl chloroformate
etc., preferably benzyl chloroformate is used.

An amount of the above-mentioned halogenoformate to be used is preferably 1.0 to 3.0 mols, more preferably 1.0 to 1.5 mols per 1 mol of the 3-hydroxyglutaric acid diester.

As the base to be used in the reaction of the present invention, an organic base such as a tertiary amine, etc. is preferably used, and there may be mentioned, for example, triethylamine, tri-n-propylamine, tri-n-butylamine, diisopropylethylamine, N-methylpiperidine, pyridine, 2,6-lutidine, 4-dimethylaminopyridine, etc., preferably pyridine, 4-dimethylaminopyridine, more preferably 4-dimethylaminopyridine is used. Incidentally, these bases may be used singly or in combination of two or more kinds.

An amount of the above-mentioned base to be used is preferably 1.0 to 3.0 mols, more preferably 1.0 to 1.5 mols per 1 mol of the 3-hydroxyglutaric acid diester.

The reaction of the present invention is carried out in the presence or absence of a solvent. As the solvent to be used, it is not specifically limited so long as it does not inhibit the reaction, and there may be mentioned, for example, aromatic hydrocarbons such as benzene, toluene, xylene, etc.; halogenated aromatic hydrocarbons such as chlorobenzene, dichlorobenzene, etc.; halogenated aliphatic hydrocarbons such as dichloromethane, chloroform, carbon tetrachloride, 1,2-dichloroethane, etc.; carboxylic acid esters such as methyl acetate, ethyl acetate, butyl acetate, etc.; amides such as N,N-dimethylformamide, N,N-dimethylacetamide, 1,3-dimethyl-2-imidazolidinone, etc.; nitriles such as acetonitrile, propionitrile, etc.; sulfoxides such as dimethylsulfoxide, etc.; ethers such as diisopropyl ether, dibutyl ether, dimethoxyethane, tetrahydrofuran, 1,4-dioxane, etc., preferably halogenated aliphatic hydrocarbons, more preferably dichloromethane, and/or 1,2-dichloroethane is/are used. Incidentally, these solvents may be used singly or in combination of two or more kinds.

An amount of the above-mentioned solvent to be used may be optionally controlled depending on a uniformity or stirrability of the reaction solution, and it is preferably 2 to 50 ml, more preferably 5 to 20 ml based on 1 g of the 3-hydroxyglutaric acid diester.

The reaction of the present invention can be carried out, for example, by mixing a 3-hydroxyglutaric acid diester, a halogenoformate, a base and a solvent in a nitrogen gas atmosphere under stirring, etc. A reaction temperature at that time is preferably -20 to 100°C, more preferably 0 to 40°C, and a reaction pressure is not specifically limited.

The 3-substituted oxyglutaric acid diester compound obtained by the reaction of the present invention is isolated and purified by a general method such as filtration, extraction, concentration, recrystallization, crystallization, column chromatography, etc., after completion of the reaction.

Next, the optically active 3-substituted oxyglutaric acid monoester compound and a process for preparing the same of the present invention are explained.

In the hydrolysis reaction for obtaining the optically active 3-substituted oxyglutaric acid monoester compound of the present invention, for example, one of the ester groups of the 3-substituted oxyglutaric acid diester (in the following, sometimes referred to as Compound (I).) represented by the above-mentioned formula (I) is selectively hydrolyzed in the presence of a hydrolase to obtain an optically active 3-substituted oxyglutaric acid monoester (in the following, it may be sometimes called to as Compound (IV).) represented by the formula (IV) as mentioned in the following formula (V): 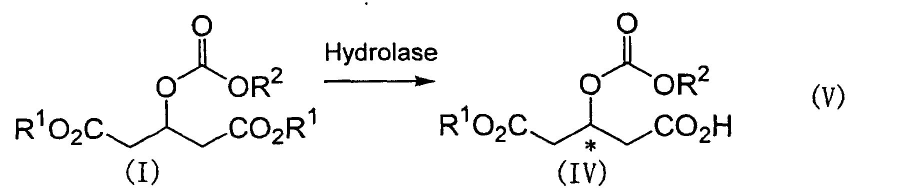 wherein R¹ and R² have the same meanings as defined above.

Compound (I) to be used in the hydrolysis reaction of the present invention is as mentioned above.

As the hydrolase to be used in the hydrolysis of the present invention, there may be mentioned, for example, protease, esterase, lipase and the like, preferably a lipase of microorganisms which are capable of isolating from yeast or bacteria, more preferably a lipase originated from *Pseudomonas* (for example, Amano PS (available from Amanoenzyme Co.), etc.), a lipase originated from *Candida antarctica* (for example, Chirazyme L-2 (available from Roche AG), etc.), particularly preferably a lipase originated from *Candida antarctica* is used. Incidentally, these hydrolases may be used in a natural form or a commercially available product as such as an immobilized enzyme, and may be used alone or in combination of two or more kinds.

An amount of the above-mentioned hydrolase to be used is preferably 0.1 to 1000 mg, more preferably 1 to 200 mg based on 1 g of Compound (I).

The hydrolysis reaction of the present invention is preferably carried out in water, in a buffer or in an aqueous inorganic base solution.

As the above-mentioned water, purified water such as deionized water, distilled water, etc. is preferably used. Incidentally, when water is used as a solvent, a weak base such as potassium hydrogen carbonate, sodium hydrogen carbonate, etc. is desirably present in the reaction system. An amount of the above-mentioned weak base to be used is preferably 0.5 to 2.0 mols, more preferably 0.5 to 1.0 mol based on 1 mol of Compound (I).

As the above-mentioned buffer, there may be mentioned, for example, an aqueous solution of an inorganic acid salt such as an aqueous sodium phosphate solution, an aqueous potassium phosphate solution, etc.; an aqueous solution of an organic acid salt such as an aqueous sodium acetate solution, an aqueous sodium citrate solution, etc. Incidentally, these aqueous solutions may be used singly or in combination of two or more kinds.

A concentration of the buffer is preferably 0.01 to 2 mol/l, more preferably 0.05 to 0.5 mol/l, and a pH of the buffer is preferably 4 to 9, more preferably 6 to 8.

As the above-mentioned aqueous inorganic base solution, there may be mentioned, for example, an aqueous solution of alkali metal carbonate such as an aqueous sodium carbonate solution, an aqueous potassium phosphate solution, etc., an aqueous solution of alkali metal hydrogen carbonate such as an aqueous sodium hydrogen carbonate solution, an aqueous potassium hydrogen carbonate solution, etc., preferably an aqueous solution of alkali metal hydrogen carbonate, more preferably an aqueous sodium carbonate solution is used. Incidentally, these aqueous inorganic base solutions may be used singly or in combination of two or more kinds.

An amount of the above-mentioned aqueous inorganic base solution to be used is preferably 1 to 5 mols, more preferably 1 to 2 mols based on 1 mol of Compound (I) in terms of the inorganic base.

An amount of the solvent (water, a buffer or an aqueous inorganic base solution) to be used in the hydrolysis reaction of the present invention is preferably 2 to 200 ml, more preferably 5 to 80 ml based on 1 g of Compound (I).

The hydrolysis reaction of the present invention can be carried out, for example, by mixing Compound (I), a hydrolase and a solvent (water, a buffer or an aqueous inorganic base solution) and reacting them under stirring, etc. A reaction temperature at that time is preferably 0 to 80°C, more preferably 10 to 50°C, and a reaction pressure is not specifically limited.

Compound (IV) obtained by the hydrolysis reaction of the present invention can be isolated and purified by, for example, extracting with an organic solvent from the reaction mixture and concentrating the extract after completion of the reaction. Incidentally, the product can be further purified by a general purification method such as crystallization, recrystallization, distillation, column chromatography, etc.

Specific examples of Compound (IV) obtained by the hydrolysis reaction of the present invention may include, for example,
optically active monomethyl 3-(R or S)-benzyloxycarbonyloxyglutarate,
optically active monoethyl 3-(R or S)-benzyloxycarbonyloxyglutarate,
optically active monopropyl 3-(R or S)-benzyloxycarbonyloxyglutarate,
optically active monobutyl 3-(R or S)-benzyloxycarbonyloxyglutarate,
optically active monopentyl 3-(R or S)-benzyloxycarbonyloxyglutarate,
optically active monohexyl 3-(R or S)-benzyloxycarbonyloxyglutarate,
optically active monoheptyl 3-(R or S)-benzyloxycarbonyloxyglutarate,
optically active monooctyl 3-(R or S)-benzyloxycarbonyloxyglutarate,
optically active monononyl 3-(R or S)-benzyloxycarbonyloxyglutarate,
optically active monodecyl 3-(R or S)-benzyloxycarbonyloxyglutarate,
optically active monoisopropyl 3-(R or S)-benzyloxycarbonyloxyglutarate,
optically active mono-s-butyl 3-(R or S)-benzyloxycarbonyloxyglutarate,
optically active mono-t-butyl 3-(R or S)-benzyloxycarbonyloxyglutarate,
optically active monochloromethyl 3-(R or S)-benzyloxycarbonyloxyglutarate,
optically active monobenzyl 3-(R or S)-benzyloxycarbonyloxyglutarate,
optically active mono-4-nitrobenzyl 3-(R or S)-benzyloxycarbonyloxyglutarate,
optically active mono-4-trifluoromethylbenzyl 3-(R or S)-benzyloxycarbonyloxyglutarate,
optically active mono-4-chlorobenzyl 3-(R or S)-benzyloxycarbonyloxyglutarate,
optically active mono-4-bromobenzyl 3-(R or S)-benzyloxycarbonyloxyglutarate,
optically active mono-4-fluorobenzyl 3-(R or S)-benzyloxycarbonyloxyglutarate,
optically active mono-4-methoxybenzyl 3-(R or S)-benzyloxycarbonyloxyglutarate,
optically active monovinyl 3-(R or S)-benzyloxycarbonyloxyglutarate,
optically active monoallyl 3-(R or S)-benzyloxycarbonyloxyglutarate,
optically active monomethyl 3-(R or S)-(2-methylbenzyl)oxycarbonyloxyglutarate,
optically active monomethyl 3-(R or S)-(3-methylbenzyl)oxycarbonyloxyglutarate,
optically active monomethyl 3-(R or S)-(4-methylbenzyl)oxycarbonyloxyglutarate,
optically active monomethyl 3-(R or S)-(2-methoxybenzyl)oxycarbonyloxyglutarate,
optically active monomethyl 3-(R or S)-(3-methoxybenzyl)oxycarbonyloxyglutarate,
optically active monomethyl 3-(R or S)-(4-methoxybenzyl)oxycarbonyloxyglutarate,
optically active monomethyl 3-(R or S)-(2-chlorobenzyl)oxycarbonyloxyglutarate,
optically active monomethyl 3-(R or S)-(3-chlorobenzyl)oxycarbonyloxyglutarate,
optically active monomethyl 3-(R or S)-(4-chlorobenzyl)oxycarbonyloxyglutarate,
optically active monomethyl 3-(R or S)-(2-bromobenzyl)oxycarbonyloxyglutarate,
optically active monomethyl 3-(R or S)-(3-bromobenzyl)oxycarbonyloxyglutarate,
optically active monomethyl 3-(R or S)-(4-bromobenzyl)oxycarbonyloxyglutarate,
optically active monomethyl 3-(R or S)-(2-fluorobenzyl)oxycarbonyloxyglutarate,
optically active monomethyl 3-(R or S)-(3-fluorobenzyl)oxycarbonyloxyglutarate,
optically active monomethyl 3-(R or S)-(4-fluorobenzyl)oxycarbonyloxyglutarate,
optically active monomethyl 3-(R or S)-(2-nitrobenzyl)oxycarbonyloxyglutarate,
optically active monomethyl 3-(R or S)-(3-nitrobenzyl)oxycarbonyloxyglutarate,
optically active monomethyl 3-(R or S)-(4-nitrobenzyl)oxycarbonyloxyglutarate,
optically active monomethyl 3-(R or S)-(2-methoxybenzyl)oxycarbonyloxyglutarate,
optically active monomethyl 3-(R or S)-(3-methoxybenzyl)oxycarbonyloxyglutarate,
optically active monomethyl 3-(R or S)-(4-methoxybenzyl)oxycarbonyloxyglutarate,
optically active monomethyl 3-(R or S)-(t-butoxybenzyl)oxycarbonyloxyglutarate,
optically active monomethyl 3-(R or S)-methoxycarbonyloxyglutarate,
optically active monomethyl 3-(R or S)-isopropoxycarbonyloxyglutarate,
optically active monomethyl 3-(R or S)-phenoxycarbonyloxyglutarate,
optically active monomethyl 3-(R or S)-vinyloxycarbonyloxyglutarate,
optically active monomethyl 3-(R or S)-allyloxycarbonyloxyglutarate,
and the like, preferably
optically active monomethyl 3-(R or S)-benzyloxycarbonyoxyglutarate, and/or
optically active monoethyl 3-(R or S)-benzyloxycarbonyoxyglutarate,
is/are used.

### Example

Next, the present invention is explained by referring to Examples but the scope of the present invention is not limited by these.

### Example 1 (Synthesis of dimethyl 3-benzyloxycarbonyloxyglutarate)

In 10 ml of 1,2-dichloromethane was dissolved 1.01 g (5.78 mmol) of dimethyl 3-hydroxyglutarate, 847 mg (6.93 mmol) of 4,4-dimethylaminopyridine and 990 µl (6.93 mmol) of benzyloxycarbonyl chloride were added to the solution at room temperature, and the mixture was reacted at 0°C for 30 minutes, and at room temperature for 1 hour under stirring. After completion of the reaction, the obtained reaction mixture was concentrated under reduced pressure, and the organic layer was extracted by adding 20 ml of ethyl acetate and 10 ml of water. The organic layer was dried over anhydrous magnesium sulfate, filtered and concentrated under reduced pressure to obtain an oily substance. The obtained oily substance was purified by silica gel column chromatography (Wakogel C-200 (trade name), n-hexane/ethyl acetate =5/1 (volume ratio)) to obtain 3-benzyloxycarbonyloxyglutarate dimethyl ester 1.30 g (Isolation yield based on 3-benzylamino-2-pentene diacid dimethyl ester=73%).

Incidentally, physical properties of the 3-benzyloxycarbonyloxyglutarate dimethyl ester were as follows.
¹H-NMR (δ (ppm), CDCl₃): 2.71-2.81 (m, 4H), 3.66 (s, 6H), 5.16 (s, 2H), 5.43 (quintet, 1H), 5.75 (m, 1H), 7.33-7.37 (m, 5H)
¹³C-NMR (δ (ppm), CDCl₃): 38.2, 51.9, 69.8, 70.7, 128.3, 128.57, 128.60, 135.1, 154.1, 170.0
MS (EI) m/z: 310 (M⁺)
MS (CI, i-C₄H₁₀) m/z: 311 (MH⁺)

| | | | | |
|---|---|---|---|---|
| Elemental analysis; | Calcd | C, | 58.05%; | H, 5.86% |
| | Found | C, | 57.43%; | H, 5.88% |

### Example 2 (Synthesis of optically active 3-(S)-benzyloxycarbonyloxyglutaric acid monomethyl ester)

To 721 mg (2.32 mmol) of dimethyl 3-benzoyloxycarbonyloxyglutarate were added 2 ml of an aqueous enzyme solution in which 72 µg of a lipase (CAL; available from Roche AG, Chirazyme L-2 (trade name)) originated from *Candida antarctica* and 195 mg (2.26 mmol) of sodium hydrogen carbonate had been dissolved, and the mixture was reacted at 30°C for 7 hours under stirring. After completion of the reaction, 10 ml of ethyl acetate was added to the obtained reaction mixture, a pH of the aqueous layer was adjusted to 1.9 by using 2 mol/L hydrochloric acid, 700 mg of sodium chloride was added to the mixture and the mixture was extracted. The obtained organic layer was dried over anhydrous magnesium sulfate, filtered and then concentrated under reduced pressure to obtain 675 mg (isolation yield based on dimethyl 3-benzyloxycarbonyloxyglutarate=98%) of an optically active monomethyl 3-(S)-benzyloxycarbonyloxyglutarate.

Incidentally, optically active monomethyl 3-(S)-benzyloxycarbonyloxyglutarate is a novel compound shown by the following physical properties.
¹H-NMR (δ (ppm), CDCl₃): 2.74-2.83 (m, 4H), 3.66 (s, 3H), 5.16 (s, 2H), 5.41 (quintet, 1H, J=6.35Hz), 7.32-7.37 (m, 5H), 9.55 (brs, 1H)
¹³C-NMR (δ (ppm), CDCl₃): 37.94, 37. 99, 52.0, 69.9, 70.4, 128.3, 128.59, 128.62, 135.0, 154.1, 170.0, 171.4, 175.4 MS (EI) m/z: 296 (M⁺)
MS (CI, i-C₄H₁₀) m/z: 297 (MH⁺)

| | | | |
|---|---|---|---|
| Elemental analysis; | Calcd | C, 56.75%; | H, 5.45% |
| | Found | C, 55.91%; | H, 5.59% |

Specific rotation: [α]²⁵_{D} -1.77° (c 2.53, CHCl₃)

### Reference example 1 (Synthesis of optically active monomethyl 3-(S)-hydroxyglutarate)

To 700 mg (1.07 mmol) of dimethyl 3-benzoyloxycarbonyloxyglutarate were added 7 ml of an aqueous enzyme solution into which 0.7 mg of a lipase (CAL; available from Roche AG, Chirazyme L-2 (trade name)) originated from *Candida Antarctica* is dissolved and 189 mg (2.26 mmol) of sodium hydrogen carbonate, and the mixture was reacted under stirring at 30°C for 2 hours. After completion of the reaction, 7 ml of ethyl acetate was added to the resulting reaction mixture, a pH of the aqueous layer was adjusted to 1.7 by using 2 mol/L hydrochloric acid, 300 mg of sodium chloride was added thereto and the organic layer was extracted. The obtained organic layer was dried, filtered, and then, concentrated under reduced pressure to obtain an oily substance. The obtained oily substance was purified by silica gel column chromatography (Wakogel C-200 (trade name), n-hexane/ethyl acetate=1/9 (volume ratio)) to obtain 655 mg (isolation yield based on dimethyl 3-benzyloxycarbonyloxyglutarate=98%) of optically active monomethyl 3-(S)-benzyloxycarbonyloxyglutarate.

Then, 655 mg of the obtained optically active monomethyl 3-(S)-benzyloxycarbonyloxyglutarate was dissolved in 6 ml of methanol. To the mixture was added 24 mg of 10% palladium/carbon powder, and the mixture was reacted under normal pressure and hydrogen atmosphere at room temperature for 2 hours under stirring. After completion of the reaction, the reaction mixture was filtered, and the filtrate was concentrated under reduced pressure to obtain 351 mg (isolation yield based on optically active monomethyl 3-(S)-benzyloxycarbonyloxyglutarate=98%) of optically active monomethyl 3-(S)-hydroxyglutarate.

Incidentally, physical properties of the optically active monomethyl 3-(S)-hydroxyglutarate were as follows.
¹H-NMR (δ (ppm), CDCl₃): 2.59 (d, 2H, J=5.86Hz), 2.60 (d, 2H, J=6.34Hz), 3.49 (s, 1H), 3.73 (s, 3H), 4.48 (quintet, 1H, 6.35), 5.49 (brs, 1H)
¹³C-NMR (δ (ppm), CDCl₃): 40.3, 40.4, 52.0, 64.6, 172.3, 176.4
MS (CI, i-C₄H₁₀) m/z: 163 (MH⁺)
Specific rotation: [α]²²_{D} +0.84° (c 4.19,CHCl₃)

Incidentally, absolute configuration was determined by comparing the specific rotation of the obtained optically active monomethyl 3-(S)-hydroxyglutarate and a sign of the specific rotation (literal value: [α]²⁵_{D} -0.43° (c 7.5, CHCl₃)) of optically active monomethyl 3-(R)-hydroxyglutarate described in Canadian Journal of Chemistry (Can. J. Chem.), 66, 1422 (1988).

### Utilizability in industry

According to the present invention, novel 3-substituted oxyglutaric acid diester compound and a process for preparing the same can be provided.

Also, according to the present invention, by a simple and easy method, an industrially suitable optically active 3-substituted oxyglutaric acid monoester compound and a process for preparing the same which can obtain an optically active 3-substituted oxyglutaric acid monoester compound from 3-substituted oxyglutaric acid diester compound (racemic mixture) with high yield and high selectivity can be provided.

## Claims

1. A 3-substituted oxyglutaric acid diester compound represented by the formula (I): 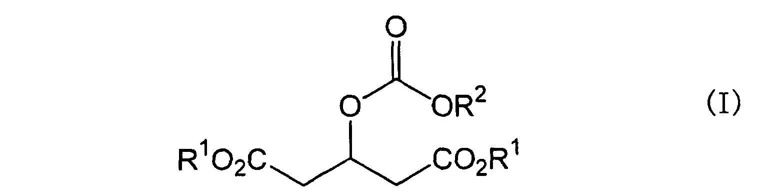 wherein R¹ may be the same or different from each other, and represents a substituted or unsubstituted alkyl group, R² represents a substituted or unsubstituted alkyl group, a substituted or unsubstituted alkenyl group, a substituted or unsubstituted aralkyl group or a substituted or unsubstituted aryl group.

2. The compound according to Claim 1, wherein R¹ is a substituted or unsubstituted alkyl group having 1 to 10 carbon atoms.

3. The compound according to Claim 1, wherein R¹ is a methyl group or an ethyl group.

4. The compound according to Claim 1, wherein R² is a group selected from the group consisting of a substituted or unsubstituted alkyl group having 1 to 10 carbon atoms, an alkenyl group having 2 to 10 carbon atoms, a substituted or unsubstituted benzyl group, a phenethyl group, a phenyl group, a naphthyl group, an anthracenyl group and a thienyl group.

5. The compound according to Claim 1, wherein R² is a group selected from the group consisting of a benzyl group, a 2-methylbenzyl group, a 3-methylbenzyl group, a 4-methylbenzyl group, a 2-methoxybenzyl group, a 3-methoxybenzyl group, a 4-methoxybenzyl group, a 2-chlorobenzyl group, a 3-chlorobenzyl group, a 4-chlorobenzyl group, a 2-bromobenzyl group, a 3-bromobenzyl group, a 4-bromobenzyl group, a 2-fluorobenzyl group, a 3-fluorobenzyl group, a 4-fluorobenzyl group, a 2-nitrobenzyl group, a 3-nitrobenzyl group, a 4-nitrobenzyl group, a 2-methoxybenzyl group, a 3-methoxybenzyl group, a 4-methoxybenzyl group, a t-butyl group, a methyl group, an isopropyl group, a phenyl group, a vinyl group and an allyl group.

6. A process for preparing a 3-substituted oxyglutaric acid diester compound according to Claim 1, which comprises reacting a 3-hydroxyglutaric acid diester represented by the formula (II): 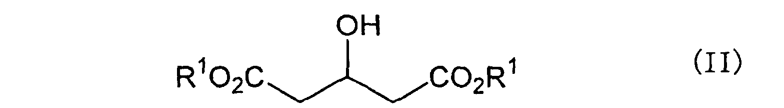 wherein R¹ may be the same or different from each other, and represents a substituted or unsubstituted alkyl group,
and a halogenoformate represented by the formula (III): 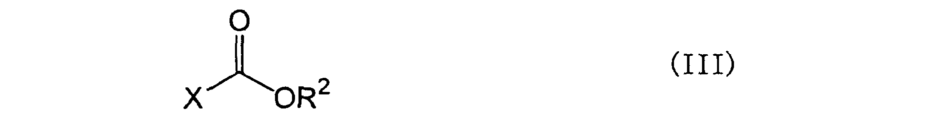 wherein X represents a halogen atom, R² represents a substituted or unsubstituted alkyl group, a substituted or unsubstituted alkenyl group, a substituted or unsubstituted aralkyl group or a substituted or unsubstituted aryl group,
in the presence of a base.

7. The process according to Claim 6, wherein the halogenoformate is benzyl chloroformate.

8. The process according to Claim 6, wherein the halogenoformate is used in an amount of 0.1 to 3.0 mols per mol of the 3-hydroxyglutaric acid diester.

9. The process according to Claim 6, wherein the base is an organic base.

10. The process according to Claim 6, wherein the organic base is a tertiary amine.

11. The process according to Claim 6, wherein the base is used in an amount of 1.0 to 3.0 mols per mol of the 3-hydroxyglutaric acid diester.

12. An optically active 3-substituted oxyglutaric acid monoester compound represented by the formula (IV): 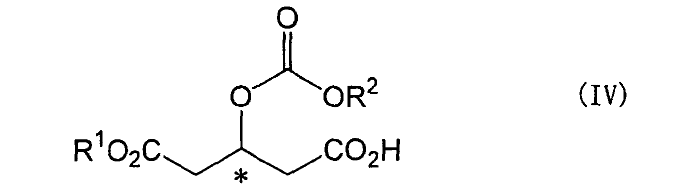 wherein R¹ represents a substituted or unsubstituted alkyl group, R² represents a substituted or unsubstituted alkyl group, a substituted or unsubstituted alkenyl group, a substituted or unsubstituted aralkyl group or a substituted or unsubstituted aryl group.

13. The compound according to Claim 12, wherein R¹ is a substituted or unsubstituted alkyl group having 1 to 10 carbon atoms.

14. The compound according to Claim 12, wherein R¹ is a methyl group or an ethyl group.

15. The compound according to Claim 12, wherein R² is a group selected from the group consisting of a substituted or unsubstituted alkyl group having 1 to 10 carbon atoms, an alkenyl group having 2 to 10 carbon atoms, a substituted or unsubstituted benzyl group, a phenethyl group, phenyl group, a naphthyl group, an anthracenyl group and a thienyl group.

16. The compound according to Claim 12, wherein R² is a group selected from the group consisting of a benzyl group, a 2-methylbenzyl group, a 3-methylbenzyl group, a 4-methylbenzyl group, a 2-methoxybenzyl group, a 3-methoxybenzyl group, a 4-methoxybenzyl group, a 2-chlorobenzyl group, a 3-chlorobenzyl group, a 4-chlorobenzyl group, a 2-bromobenzyl group, a 3-bromobenzyl group, a 4-bromobenzyl group, a 2-fluorobenzyl group, a 3-fluorobenzyl group, a 4-fluorobenzyl group, a 2-nitrobenzyl group, a 3-nitrobenzyl group, a 4-nitrobenzyl group, a 2-methoxybenzyl group, a 3-methoxybenzyl group, a 4-methoxybenzyl group, a t-butyl group, a methyl group, an isopropyl group, a phenyl group, a vinyl group and an allyl group.

17. A process for preparing an optically active 3-substituted oxyglutaric acid monoester compound according to Claim 12, which comprises selectively hydrolyzing one of ester groups of a 3-substituted oxyglutaric acid diester compound represented by the formula (I): 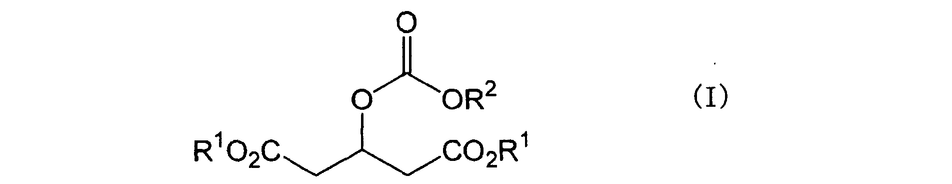 wherein R¹ represents a substituted or unsubstituted alkyl group, R² represents a substituted or unsubstituted alkyl group, a substituted or unsubstituted alkenyl group, a substituted or unsubstituted aralkyl group or a substituted or unsubstituted aryl group,
in the presence of a hydrolase.

18. The preparation process according to Claim 17, wherein the hydrolase is a protease, an esterase or a lipase.

19. The preparation process according to Claim 17, wherein the hydrolase is a lipase originated from *Candida antarctica.*

20. The preparation process according to Claim 17, wherein the hydrolysis is carried out in water, in a buffer or in an aqueous inorganic base solution.
